# EUROPEAN PATENT SPECIFICATION

(11) **EP 1 891 973 B1**
(45) Date of publication and mention of the grant of the patent: **21.07.2010**
(21) Application number: 07019644.9
(22) Date of filing: 31.05.2005
(51) Int. Cl.: A61K 45/00, A61K 31/439, A61K 31/167, A61K 31/137, A61P 11/00, A61P 11/06, A61P 11/08

(54) **Combinations comprising antimuscarinic agents and PDE4 inhibitors**
Kombinationen mit Antimuskarin-Wirkstoffen und PDE4-Hemmern
Combinaisons comportant des agents antimuscarinique et inhibiteurs PDE4

(30) Priority: 31.05.2004 ES 200401312
(43) Date of publication of application: 27.02.2008
(62) Divisional of application: 05747758.0
(73) Proprietor: Almirall, S.A., 08022 Barcelona (ES)
(72) Inventor: Gras Escardo, Jordi, 08018 Barcelona (ES); Llenas Calvo, Jesus, 08021 Barcelona (ES); Ryder, Hamish, 08190 La Floresta, Sant Cugat del Vallés (Barcelona) (ES); Orviz Diaz, Pio, 08198 Sant Cugat del Vallés (Barcelona) (ES)
(74) Representative: Srinivasan, Ravi Chandran

(56) References cited:
- WO-A-01/04118
- WO-A-02/053564
- WO-A-02/096423
- WO-A-03/087094
- WO-A-2004/084896
- WO-A-2004/084897

## Description

The present invention relates to new combinations of certain antimuscarinic agents with PDE4 inhibitors and their use in the treatment of respiratory disorders.

### BACKGROUND OF THE INVENTION

PDE4 inhibitors and antimuscarinic agents, in particular antagonists of M3 muscarinic receptors, are two classes of drugs useful in the treatment of respiratory disorders such as, asthma or Chronic Obstructive Pulmonary Diseases (COPD).

Although PDE4 inhibitors and antimuscarinic agents may be effective therapies, there exists a clinical need for asthma and COPD therapies having potent and selective action and having an advantageous profile of action.

It is known that both classes of drugs can be used in combination

Combinations of drugs in which the active ingredients operate via different physiological pathways are known to be therapeutically useful. Frequently, the therapeutic advantage arises because the combination can achieve a therapeutically useful effect using lower concentrations of each active component. This enables the side-effects of the medication to be minimised. Thus, the combination can be formulated so that each active ingredient is present at a concentration which is subclinical in cells other than the target disease cells. The combination is nevertheless therapeutically effective in target cells which respond to both ingredients.

### DESCRIPTION OF THE INVENTION

Surprisingly, an unexpectedly beneficial therapeutic effect can be observed in the treatment of inflammatory or obstructive diseases of the respiratory tract if an antimuscarinic of formula (I) used with one or more PDE4 inhibitors. In view of this effect the pharmaceutical combinations according to the invention can be used in smaller doses than would be the case with the individual compounds used in monotherapy in the usual way. This reduces unwanted side effects such as may occur when PDE4 inhibitors or antimuscarinics of formula (I) are administered alone.

The present invention accordingly provides a combination which comprises (a) a PDE4 inhibitor and (b) an antagonist of M3 muscarinic receptors which is 3(R)-(2-hydroxy-2,2-dithien-2-ylacetoxy)-1-(3-phenoxypropyl)-1-azoniabicyclo[2.2.2]octane, in the form of a salt having an anion X, which is a pharmaceutically acceptable anion of a mono or polyvalent acid.

Examples of pharmaceutically acceptable anions of mono or polyvalent acids are the anions derived from inorganic acids such as hydrochloric acid, hydrobromic acid, sulphuric acid, phosphoric acid or organic acids such as methanosulphonic acid, acetic acid, fumaric acid, succinic acid, lactic acid, citric acid or maleic acid. Furthermore, mixtures of the aforementioned acids can be used.

Typically the antagonist of M3 muscarinic receptors is 3(R)-(2-hydroxy-2,2-dithien-2-ylacetoxy)-1-(3-phenoxypropyl)-1-azoniabicyclo[2.2.2]octane bromide.

Typically the combination contains the active ingredients (a) and (b) forming part of a single pharmaceutical composition.

For the avoidance of doubt, the term 3(R)-(2-hydroxy-2,2-dithien-2-ylacetoxy)-1-(3-phenoxypropyl)-1-azoniabicyclo[2.2.2]octane is meant to embrace the salts in dissociated, partially dissociated or undissociated form, for example in aqueous solution. The different salts of the compound may exist in the form of solvates, i.e. in the form of hydrates and all these forms are also within the scope of the present invention. Furthermore the different salts and solvates of the compound may exist in amorphous form or in the form of different polymorphs within the scope of the present invention.

Also provided is a product comprising (a) a PDE4 inhibitor and (b) an M3 antagonist of the invention as a combined preparation for simultaneous, separate or sequential use in the treatment of a human or animal patient. Typically the product is for simultaneous, separate or sequential use in the treatment of a respiratory disease which is asthma, acute or chronic bronchitis, emphysema, chronic obstructive pulmonary disease (COPD), bronchial hyperreactivity or rhinitis

The present invention further provides the use of (a) a PDE4 inhibitor and (b) an M3 antagonist of the invention for the preparation of a medicament for simultaneous, concurrent, separate or sequential use in the treatment of a said respiratory disease in a human or animal patient.

Also provided is the use of (b) an M3 antagonist of the invention for the preparation of a medicament, for simultaneous, concurrent, separate or sequential use in combination with (a) a PDE4 inhibitor for the treatment of a said respiratory disease in a human or animal patient.

Also provided is the use of (a) a PDE4 inhibitor for the preparation of a medicament for use in the treatment of a said respiratory disease in a human or animal patient by simultaneous, concurrent, separate or sequential coadministration with (b) an M3 antagonist of the invention.

Typically said respiratory disease is asthma or chronic obstructive pulmonary disease (COPD).

Preferably said patient is human.

Also provided is a pharmaceutical composition comprising (a) a PDE4 inhibitor; and (b) an M3 antagonist of the invention in association with (c) a pharmaceutically acceptable carrier or diluent.

The invention also provides a kit of parts comprising (b) an M3 antagonist of the invention together with instructions for simultaneous, concurrent, separate or sequential use in combination with (a) a PDE4 inhibitor for the treatment of a human or animal patient suffering from or susceptible to a said respiratory disease.

Further provided is a package comprising (b) an M3 antagonist of the invention and (a) a PDE4 inhibitor for the simultaneous, concurrent, separate or sequential use in the treatment of a said respiratory disease.

Further provided is a combination, product, kit of parts or package as hereinabove described wherein such combination, product, kit of parts or package further comprises (c) another active compound selected from: (a) β2 agonist, (b) cortiocosteroids, (c) leukotriene D4 antagonists, (d) inhibitors of egfr-kinase, (e) p38 kinase inhibitors and (f) NK1 receptor agonists for simultaneous, separate or sequential use. Typically the additional active compound (c) is selected from the group consisting of (a) β2 agonists and (b) cortiocosteroids.

It is an embodiment of the present invention that the combination, product, kit of parts or package comprise (b) an M3 antagonist of the invention and (a) a PDE4 inhibitor as the sole active compounds.

A further embodiment of the present invention is the use of b) an M3 antagonist of the invention and (a) a PDE4 inhibitor without any other active compound for the preparation of a medicament for simultaneous, concurrent, separate or sequential use in the treatment of a said respiratory disease in a human or animal patient.

Examples of PDE4 inhibitors to be used in the combinations of the present invention are selected from the group comprising Theophylline, Drotaverine hydrochloride, Cilomilast, Roflumilast, Denbufylline, Rolipram, Tetomilast, Enprofylline, Arofylline, Cipamfylline, Tofimilast, Filaminast, Piclamilast, (R)-(+)-4-[2-(3-Cyclopentyloxy-4-methoxyphenyl)-2-phenylethyl]pyridine, Mesopram, N-(3,5-Dichloro-4-pyridinyl)-2-[1-(4-fluorobenzyl)-5-hydroxy-1H-indol-3-yl]-2-oxoacetamide, CDC-801 (ex. Celgene), CC-1088 (ex. Celgene), Lirimilast, ONO-6126 (ex. Ono), CC-10004 (ex. Celgene), MN-001 (ex. Kyorin), KW-4490 (ex. Kyowa Hakko), Benafentrine dimaleate, Zardaverine, Tolafentrine, 3-[3-(Cyclopentyloxy)-4-methoxybenzyl]-6-(ethylamino)-8-isopropyl-3H-purine hydrochloride, N-(3,5-Dichloro-4-pyridinyl)-8-methoxyquinoline-5-carboxamide, 4-(3-Chlorophenyl)-1,7-diethylpyrido[2,3-d]pyrimidin-2(1H)-one, N-[9-Methyl-4-oxo-1-phenyl-3,4,6,7-tetrahydropyrrolo[3,2,1-jk][1,4]benzodiazepin-3(R)-yl]pyridine-4-carboxamide, 3,5-Dichloro-4-[8-methoxy-2-(trifluoromethyl)quinolin-5-ylcarboxamido]pyridine-1-oxide, NIK-616 (ex. Nikken Chemicals), CDC-998 (ex. Celgene), Project PDE 4 (ex. Celltech), EHT-0202 (ex. ExonHit Therapeutics), 5(S)-[3-(Cyclopentyloxy)-4-methoxyphenyl]-3(S)-(3-methylbenzyl)piperidin-2-one, ND-1251 (ex. Neuro3d), GRC-3886 (ex. Glenmark Pharmaceuticals), Atizoram, Pumafentrine, 4-[6,7-Diethoxy-2,3-bis(hydroxymethyl)naphthalen-1-yl]-1-(2-methoxyethyl)pyridin-2(1H)-one, 2-[4-[6,7-Diethoxy-2,3-bis(hydroxymethyl)naphthalen-1-yl]pyridin-2-yl]-4-(3-pyridyl)phthalazin-1(2H)-one hydrochloride, 1-Ethyl-8-methoxy-3-methyl-5-propylimidazo[1,5-a]pyrido[3,2-e]pyrazin-4(5H)-one, 4-(3-Bromophenyl)-1-ethyl-7-methyl-1,8-naphthyridin-2(1H)-one, N-[9-Amino-4-oxo-1-phenyl-3,4,6,7-tetrahydropyrrolo[3,2,1-jk][1,4]benzodiazepin- 3(R)-yl]pyridine-3-carboxamide, hydroxypumafentrine and the compounds exemplified in PCT patent applications number WO 03/097613, WO, 2004/058729 and WO 2005/

Preferred PDE4 inhibitors under the present invemtion are: Theophylline, Drotaverine hydrochloride, Cilomilast, Roflumilast, Denbufylline, Rolipram, Tetomilast, Enprofylline, Arofylline, Cipamfylline, Tofimilast, Filaminast, Piclamilast, (R)-(+)-4-[2-(3-Cyclopentyloxy-4-methoxyphenyl)-2-phenylethyl]pyridine, Mesopram, N-(3,5-Dichloro-4-pyridinyl)-2-[1-(4-fluorobenzyl)-5-hydroxy-1H-indol-3-yl]-2-oxoacetamide, CDC-801 (ex. Celgene), CC-1088 (ex. Celgene), Lirimilast, ONO-6126 (ex. Ono), CC-10004 (ex. Celgene), MN-001 (ex. Kyorin) and the compounds exemplified in PCT patent applications number WO 03/097613, WO, 2004/058729 and WO 2005/.

Still more preferred PDE4 inhibitors under the present invention are : Theophylline, Drotaverine hydrochloride, Cilomilast, Roflumilast, Denbufylline, Rolipram, Tetomilast, N-(3,5-Dichloro-4-pyridinyl)-2-[1-(4-fluorobenzyl)-5-hydroxy-1H-indol-3-yl]-2-oxoacetamide, Enprofylline, Arofylline and the compounds exemplified in PCT patent applications number WO 03/097613, WO, 2004/058729 and WO 2005/. The most preferred PDE4 inhibitors are Cilomilast, Roflumilast, Denbufylline, Tetomilast and the compounds exemplified in PCT patent applications number WO 03/097613, WO, 2004/058729 and WO 2005/, in special Cilomilast, Roflumilast, Denbufylline, and Tetomilast most preferably Roflumilast and Cilomilast.

Pharmaceutically acceptable salt forms of the combinations of compounds of the present invention are prepared for the most part by conventional means. Where the component compound contains a carboxylic acid group, a suitable salt thereof may be formed by reacting the compound with an appropriate base to provide the corresponding base addition salt. Examples of such bases are alkali metal hydroxides including potassium hydroxide, sodium hydroxide, and lithium hydroxide; alkaline earth metal hydroxides such as barium hydroxide and calcium hydroxide; alkali metal alkoxides, e.g., potassium ethanolate and sodium propanolate, and various organic bases such as piperidine, diethanolamine, and N-methylglutamine. Also included are the aluminum salts of the component compounds of the present invention.

For certain component compounds, acid addition salts may be formed by treating said compounds with pharmaceutically acceptable organic and inorganic acids, e.g., hydrohalides such as hydrochloride, hydrobromide, hydroiodide; other mineral acids and their corresponding salts such as sulfate, nitrate, phosphate, etc.; and alkyl- and monoarylsulfonates such as ethanesulfonate, toluenesulfonate, and benzenesulfonate; and other organic acids and their corresponding salts such as acetate, tartrate, maleate, succinate, citrate, benzoate, salicylate, ascorbate, etc.

Accordingly, the pharmaceutically acceptable acid addition salts of the component compounds of the present invention include, but are not limited to: acetate, adipate, alginate, arginate, aspartate, benzoate, benzenesulfonate (besylate), bisulfate, bisulfite, bromide, butyrate, camphorate, camphorsulfonate, caprylate, chloride, chlorobenzoate, citrate, cyclopentanepropionate, digluconate, dihydrogenphosphate, dinitrobenzoate, dodecylsulfate, ethanesulfonate, fumarate, galacterate (from mucic acid), galacturonate, glucoheptanoate, gluconate, glutamate, glycerophosphate, hemisuccinate, hemisulfate, heptanoate, hexanoate, hippurate, hydrochloride, hydrobromide, hydroiodide, 2-hydroxyethanesulfonate, iodide, isethionate, isobutyrate, lactate, lactobionate, malate, maleate, malonate, mandelate, metaphosphate, methanesulfonate, methylbenzoate, monohydrogenphosphate, 2-naphthalenesulfonate, nicotinate, nitrate, oxalate, oleate, pamoate, pectinate, persulfate, phenylacetate, 3-phenylpropionate, phosphate, phosphonate, and phthalate.

Particularly preferred examples of pharmacologically acceptable acid addition salts of the PDE4 inhibitors are the pharmaceutically acceptable salts which are selected from among the salts of hydrochloric acid, hydrobromic acid, sulfuric acid, phosphoric acid, methanesulfonic acid, acetic acid, fumaric acid, succinic acid, lactic acid, citric acid, tartaric acid, 1-hydroxy-2-naphthalenecarboxylic acid, or maleic acid. If desired, mixtures of the abovementioned acids may also be used to prepare the salts of the PDE4 inhibitors.

In the pharmaceutical compositions according to the invention, the PDE4 inhibitors may be present in the form of their racemates, enantiomers or mixtures thereof. The separation of the enantiomers from the racemates may be carried out using methods known in the art (e.g., by chromatography on chiral phases, etc.).

A preferred embodiment of the present invention is a combination of an M3 antagonist of the invention with a PDE4 inhibitor selected from Cilomilast, Roflumilast, Denbufylline, and Tetomilast. Even more preferred is the combination of an M3 antagonist of the invention with cilomilast and the combination of an M3 antagonist of the invention with roflumilast.

Another embodiment of the present invention is a combination of an M3 antagonist which is 3(R)-(2-hydroxy-2,2-dithien-2-ylacetoxy)-1-(3-phenoxypropyl)-1-azoniabicyclo[2.2.2]octane bromide with a PDE4 inhibitor selected from cilomilast, roflumilast, denbufylline, and tetomilast.

According to one embodiment of the invention the PDE4 inhibitor is roflumilast.

According to another embodiment of the invention the PDE4 inhibitor is cilomilast.

The combinations of the invention can optionally comprise one or more additional active substances which are known to be useful in the treatment of respiratory disorders, such as β2-agonists, corticosteroids or glucocorticoids, leukotriene D4 inhibitors, inhibitors of egfr-kinase, p38 kinase inhibitors and/or NK1-receptor antagonists.

The preferred β2-agonists to be used in the combinations of the invention are: arformoterol, bambuterol, bitolterol, broxaterol, carbuterol, clenbuterol, dopexamine, fenoterol, formoterol, hexoprenaline, ibuterol, isoetharine, isoprenaline, levosalbutamol, mabuterol, meluadrine, metaprotenerol, nolomirole, orciprenaline, pirbuterol, procaterol, reproterol, ritodrine, rimoterol, salbutamol, salmefamol, salmeterol, sibenadet, sotenerot, sulfonterol, terbutaline, tiaramide, tulobuterol, GSK-597901, GSK-159797, GSK-678007, GSK-642444, GSK-159802, HOKU-81, (-)-2-[7(S)-[2(R)-Hydroxy-2-(4-hydroxyphenyl)ethylamino]-5,6,7,8-tetrahydro-2-naphthyloxy]-N,N-dimethylacetamide hydrochloride monohydrate, carmoterol, QAB-149 and 5-[2-(5,6-diethylindan-2-ylamino)-1-hydroxyethyl]-8-hydroxy-1H-quinolin-2-one, 4-hydroxy-7-[2-{[2-{[3-(2-phenylethoxy)propyl]sulfonyl} ethyl]amino}ethyl]-2(3H)-benzothiazolone, 1-(2-fluoro-4-hydroxyphenyl)-2-[4-(1-benzimidazolyl)-2-methyl-2-butylamino]ethanol, 1-[3-(4-methoxybenzylamino)-4-hydroxyphenyl]-2-[4-(1-benzimidazolyl)-2-methyl-2-butylamino]ethanol, 1-[2H-5-hydroxy-3-oxo-4H-1,4-benzoxazin-8-yl]-2-[3-(4-N,N -dimethylaminophenyl)-2-methyl-2-propylamino]ethanol, 1-[2H-5-hydroxy-3-oxo-4H-1,4-benzoxazin-8-yl]-2-[3-(4-methoxyphenyl)-2-methyl-2-propylamino]ethanol, 1-[2H-5-hydroxy-3-oxo-4H-1,4-benzoxazin-8-yl]-2-[3-(4-n-butyloxyphenyl)-2-methyl-2-propylamino]ethanol, 1-[2H-5-hydroxy-3-oxo-4H-1,4-benzoxazin-8-yl]-2-{4-[3-(4-methoxyphenyl)-1,2,4-triazol-3-yl]-2-methyl-2-butylamino}ethanol, 5-hydroxy-8-(1-hydroxy-2-isopropylaminobutyl)-2H-1,4-benzoxazin-3-(4H)-one, 1-(4-amino-3-chloro-5-trifluoromethylphenyl)-2-tert-butylamino)ethanol and 1-(4-ethoxycarbonylamino-3-cyano-5-fluorophenyl)-2-(tert-butylamino)ethanol optionally in the form of their racemates, their enantiomers, their diastereomers, and mixtures thereof, and optionally their pharmacologically-compatible acid addition salts.

Examples of suitable corticosteroids and glucocorticoids that can be combined with M3-antagonists and PDE4 inhibitors are prednisolone, methylprednisolone, dexamethasone, naflocort, deflazacort, halopredone acetate, budesonide, beclomethasone dipropionate, hydrocortisone, triamcinolone acetonide, fluocinolone acetonide, fluocinonide, clocortolone pivalate, methylprednisolone aceponate, dexamethasone palmitoate, tipredane, hydrocortisone aceponate, prednicarbate, alclometasone dipropionate, halometasone, methylprednisolone suleptanate, mometasone furoate, rimexolone, prednisolone farnesylate, ciclesonide, deprodone propionate, fluticasone propionate, halobetasol propionate, loteprednol etabonate, betamethasone butyrate propionate, flunisolide, prednisone, dexamethasone sodium phosphate, triamcinolone, betamethasone 17-valerate, betamethasone, betamethasone dipropionate, hydrocortisone acetate, hydrocortisone sodium succinate, prednisolone sodium phosphate and hydrocortisone probutate.

Examples of suitable LTD4 antagonists that can be combined with M3 antagonists and PDE4 inhibitors are tomelukast, lbudilast, pobilukast, pranlukast hydrate, zafirlukast, ritolukast, verlukast, sulukast, cinalukast, iralukast sodium, montelukast sodium, 4-[4-[3-(4-Acetyl-3-hydroxy-2-propylphenoxy)propylsulfonyl]phenyl]-4-oxobutyric acid, [[5-[[3-(4-Acetyl-3-hydroxy-2-propylphenoxy)propyl]thio]-1 ,3,4-thiadiazol-2-yl]thio]acetic acid, 9-[(4-Acetyl-3-hydroxy-2-n-propylphenoxy)methyl]-3-(1H-tetrazol-5-yl)-4H-pyrido[1,2-a]pyrimidin-4-one, 5-[3-[2-(7-Chloroquinolin-2-yl)vinyl]phenyl]-8-(N,N-dimethylcarbamoyl)-4,6-dithiaoctanoic acid sodium salt; 3-[1-[3-[2-(7-Chloroquinolin-2-yl)vinyl]phenyl]-1-[3-(dimethylamino)-3-oxopropylsulfanyl]methylsulfanyl]propionic acid sodium salt, 6-(2-Cyclohexylethyl)-[1,3,4]thiadiazolo[3,2-a]-1,2,3-triazolo[4,5-d]pyrimidin-9(1H)-one, 4-[6-Acetyl-3-[3-(4-acetyl-3-hydroxy-2-propylphenylthio)propoxy]-2-propylphenoxy]butyric acid, (R)-3-Methoxy-4-[1-methyl-5-[N-(2-methyl-4,4,4-trifluorobutyl)carbamoyl]indol-3-ylmethyl]-N-(2-methylphenylsulfonyl)benzamide, (R)-3-[2-Methoxy-4-[N-(2-methylphenylsulfonyl)carbamoyl]benzyl]-1-methyl-N-(4,4,4-trifluoro-2-methylbutyl)indole-5-carboxamide, (+)-4(S)-(4-Carboxyphenylthio)-7-[4-(4-phenoxybutoxy)phenyl]-5(Z)-heptenoic acid and the compounds claimed in the PCT patent application number PCT/EP03/12581.

Examples of suitable inhibitors of egfr-kinase that can be combined with M3 antagonists and PDE4 inhibitors are palifermin, cetuximab, gefitinib, repifermin, erlotinib hydrochloride, canertinib dihydrochloride, lapatinib, and N-[4-(3-Chloro-4-fluorophenylamino)-3-cyano-7-ethoxyquinolin-6-yl]-4-(dimethylamino)-2(E)-butenamide.

Examples of suitable p38 kinase inhibitors that can be combined with M3 antagonists and PDE4 inhibitors are chlormethiazole edisylate, doramapimod, 5-(2,6-Dichlorophenyl)-2-(2,4-difluorophenylsulfanyl)-6H-pyrimido[3,4-b]pyridazin-6-one, 4-Acetamido-N-(tert-butyl)benzamide, SCIO-469 (described in Clin Pharmacol Ther 2004, 75(2): Abst PII-7 and VX-702 described in Circulation 2003, 108(17, Suppl. 4): Abst 882.

Examples of suitable NK1-receptor antagonists that can be combined with M3 antagonists and PDE4 inhibitors are nolpitantium besilate, dapitant, lanepitant, vofopitant hydrochloride, aprepitant, ezlopitant, N-[3-(2-Pentylphenyl)propionyl]-threonyl-N-methyl-2,3-dehydrotyrosyl-leucyl-D-phenylalanyl-allo-threonyl-asparaginyl-serine C-1.7-O-3.1 lactone, 1-Methylindol-3-ylcarbonyl-[4(R)-hydroxy]-L-prolyl-[3-(2-naphthyl)]-L-alanine N-benzyl-N-methylamide, (+)-(2S,3S)-3-[2-Methoxy-5-(trifluoromethoxy)benzylamino]-2-phenylpiperidine, (2R,45)-N-[1-[3,5-Bis(trifluoromethyl)benzoyl]-2-(4-chlorobenzyl)piperidin-4-yl]quinoline-4-carboxamide, 3-[2(R)-[1(R)-[3,5-Bis(trifluoromethyl)phenyl]ethoxy]-3(S)-(4-fluorophenyl)morpholin-4-ylmethyl]-5-oxo-4,5-dihydro-1H-1,2,4-triazole-1-phosphinic acid bis(N-methyl-D-glucamine) salt; [3-[2(R)-[1(R)-[3,5-bis(trifluoromethyl)phenyl]ethoxy]-3(S)-(4-fluorophenyl)-4-morpholinylmethyl]-2,5-dihydro-5-oxo-1H-1,2,4-triazol-1-yl]phosphonic acid 1-deoxy-1-(methylamino)-D-glucitol (1:2) salt, 1'-[2-[2(R)-(3,4-Dichlorophenyl)-4-(3,4,5-trimethoxybenzoyl)morpholin-2-yl]ethyl]spiro[benzo[c]thiophen-1(3H)-4'-piperidine] 2(S)-oxide hydrochloride and the compound CS-003 described in Eur Respir J 2003, 22(Suppl. 45): Abst P2664.

The active compounds in the combination, i.e. the M3 antagonist of the invention, the PDE4 inhibitors and any other optional active compounds may be administered together in the same pharmaceutical composition or in different compositions intended for separate, simultaneous, concomitant or sequential administration by the same or a different route.

In a preferred embodiment of the invention the active compounds in the combination are administered by inhalation through a common delivery device, wherein they can be formulated in the same or in different pharmaceutical compositions.

In the most preferred embodiment the M3 antagonist of the invention and the PDE4 inhibitor are both present in the same pharmaceutical composition and are administered by inhalation through a common delivery device.

In one aspect the invention provides a combination as herein defined **characterised in that** the active ingredients (a) and (b) form part of a single pharmaceutical composition.

A said pharmaceutical composition can be prepared by mixing and processing an M3 antagonist of the invention, a PDE4 inhibitor and optionally other additives and/or carriers by methods known per se.

The active compounds in the combination, i.e. the M3 antagonist of the invention, the PDE4 inhibitor and any other optional active compounds may be administered by any suitable route, depending on the nature of the disorder to be treated, e.g. orally (as syrups, tablets, capsules, lozenges, controlled-release preparations, fast-dissolving preparations, lozenges, etc); topically (as creams, ointments, lotions, nasal sprays or aerosols, etc); by injection (subcutaneous, intradermic, intramuscular, intravenous, etc.) or by inhalation (as a dry powder, a solution, a dispersion, etc).

The pharmaceutical formulations may conveniently be presented in unit dosage form and may be prepared by any of the methods well known in the art of pharmacy. All methods include the step of bringing the active ingredient(s) into association with the carrier. In general the formulations are prepared by uniformly and intimately bringing into association the active ingredient with liquid carriers or finely divided solid carriers or both and then, if necessary, shaping the product into the desired formulation.

Formulations of the present invention suitable for oral administration may be presented as discrete units such as capsules, cachets or tablets each containing a predetermined amount of the active ingredient; as a powder or granules; as a solution or a suspension in an aqueous liquid or a non-aqueous liquid; or as an oil- in-water liquid emulsion or a water-in-oil liquid emulsion. The active ingredient may also be presented as a bolus, electuary or paste.

A syrup formulation will generally consist of a suspension or solution of the compound or salt in a liquid carrier for example, ethanol, natural, synthetic or semisynthetic oils such as peanut oil and olive oil, glycerine or water with flavouring, sweetener and/or colouring agent.

Where the composition is in the form of a tablet, any pharmaceutical carrier routinely used for preparing solid formulations may be used. Examples of such carriers include celluloses, stearates such as magnesium stearate or stearic acid, talc, gelatine, acacia, starches, lactose and sucrose.

A tablet may be made by compression or moulding, optionally with one or more accessory ingredients. Compressed tablets may be prepared by compressing in a suitable machine the active ingredient in a free-flowing form such as a powder or granules, optionally mixed with binders, lubricants, inert diluents, lubricating, surface active or dispersing agents. Moulded tablets may be made by moulding in a suitable machine a mixture of the powdered blend comprising the active compounds moistened with an inert liquid diluent and optionally dried and sieved. The tablets may optionally be coated or scored and may be formulated so as to provide modified (i.e. slow or controlled) release of the active ingredient therein.

Where the composition is in the form of a capsule, any routine encapsulation is suitable, for example using the aforementioned carriers in a hard gelatine capsule. Where the composition is in the form of a soft gelatine capsule any pharmaceutical carrier routinely used for preparing dispersions or suspensions may be considered, for example aqueous gums, celluloses, silicates or oils, and are incorporated in a soft gelatine capsule.

Dry powder compositions for topical delivery to the lung by inhalation may, for example, be presented in different primary packaging systems (such as capsules and cartridges of for example gelatine or blisters of for example laminated aluminium foil), for use in an inhaler or insufflator.

Packaging of the formulation may be suitable for unit dose or multi-dose delivery. In the case of multi- dose delivery, the formulation can be pre-metered or metered in use. Dry powder inhalers are thus classified into three groups: (a) single dose, (b) multiple unit dose and (c) multi dose devices.

Formulations generally contain a powder mix for inhalation of the compounds of the invention and a suitable powder base (carrier substance) such as lactose or starch. Use of lactose is preferred. Each capsule or cartridge may generally contain between 2µg and 400 µg of each therapeutically active ingredient. Alternatively, the active ingredient (s) may be presented without excipients.

For single dose inhalers of the first type, single doses have been weighed by the manufacturer into small containers, which are mostly hard gelatine capsules. A capsule has to be taken from a separate box or container and inserted into a receptacle area of the inhaler. Next, the capsule has to be opened or perforated with pins or cutting blades in order to allow part of the inspiratory air stream to pass through the capsule for powder entrainment or to discharge the powder from the capsule through these perforations by means of centrifugal force during inhalation. After inhalation, the emptied capsule has to be removed from the inhaler again. Mostly, disassembling of the inhaler is necessary for inserting and removing the capsule, which is an operation that can be difficult and burdensome for some patients. Other drawbacks related to the use of hard gelatine capsules for inhalation powders are (a) poor protection against moisture uptake from the ambient air, (b) problems with opening or perforation after the capsules have been exposed previously to extreme relative humidity, which causes fragmentation or indenture, and (c) possible inhalation of capsule fragments. Moreover, for a number of capsule inhalers, incomplete expulsion has been reported (e. g. Nielsen et al, 1997).

Some capsule inhalers have a magazine from which individual capsules can be transferred to a receiving chamber, in which perforation and emptying takes place, as described in WO 92/03175. Other capsule inhalers have revolving magazines with capsule chambers that can be brought in line with the air conduit for dose discharge (e. g. WO91/02558 and GB 2242134). They comprise the type of multiple unit dose inhalers together with blister inhalers, which have a limited number of unit doses in supply on a disk or on a strip.

Blister inhalers provide better moisture protection of the medicament than capsule inhalers. Access to the powder is obtained by perforating the cover as well as the blister foil, or by peeling off the cover foil. When a blister strip is used instead of a disk, the number of doses can be increased, but it is inconvenient for the patient to replace an empty strip. Therefore, such devices are often disposable with the incorporated dose system, including the technique used to transport the strip and open the blister pockets.

Multi-dose inhalers do not contain pre-measured quantities of the powder formulation. They consist of a relatively large container and a dose measuring principle that has to be operated by the patient. The container bears multiple doses that are isolated individually from the bulk of powder by volumetric displacement. Various dose measuring principles exist, including rotatable membranes (e. g. EP0069715) or disks (e. g. GB 2041763; EP 0424790; DE 4239402 and EP 0674533), rotatable cylinders (e. g. EP 0166294; GB 2165159 and WO 92/09322) and rotatable frustums (e. g. WO 92/00771), all having cavities which have to be filled with powder from the container. Other multi dose devices have measuring slides (e. g.US 5201308 and WO 97/00703) or measuring plungers with a local or circumferential recess to displace a certain volume of powder from the container to a delivery chamber or an air conduit e. g. EP 0505321, WO 92/04068 and WO 92/04928.

Reproducible dose measuring is one of the major concerns for multi dose inhaler devices.

The powder formulation has to exhibit good and stable flow properties, because filling of the dose measuring cups or cavities is mostly under the influence of the force of gravity.

For reloaded single dose and multiple unit dose inhalers, the dose measuring accuracy and reproducibility can be guaranteed by the manufacturer. Multi dose inhalers on the other hand, can contain a much higher number of doses, whereas the number of handlings to prime a dose is generally lower.

Because the inspiratory air stream in multi-dose devices is often straight across the dose measuring cavity, and because the massive and rigid dose measuring systems of multi dose inhalers can not be agitated by this inspiratory air stream, the powder mass is simply entrained from the cavity and little de-agglomeration is obtained during discharge.

Consequently, separate disintegration means are necessary. However in practice, they are not always part of the inhaler design. Because of the high number of doses in multi- dose devices, powder adhesion onto the inner walls of the air conduits and the de- agglomeration means must be minimized and/or regular cleaning of these parts must be possible, without affecting the residual doses in the device. Some multi dose inhalers have disposable drug containers that can be replaced after the prescribed number of doses has been taken (e. g. WO 97/000703). For such semi-permanent multi dose inhalers with disposable drug containers, the requirements to prevent drug accumulation are even stricter.

Apart from applications through dry powder inhalers the compositions of the invention can be administered in aerosols which operate via propellant gases or by means of so-called atomisers, via which solutions of pharmacologically-active substances can be sprayed under high pressure so that a mist of inhalable particles results. The advantage of these atomisers is that the use of propellant gases can be completely dispensed with.

Such atomisers are described, for example, in PCT Patent Application No. W0 91/14468 and International Patent Application No. WO 97/12687, reference here being made to the contents thereof.

Spray compositions for topical delivery to the lung by inhalation may for example be formulated as aqueous solutions or suspensions or as aerosols delivered from pressurised packs, such as a metered dose inhaler, with the use of a suitable liquefied propellant. Aerosol compositions suitable for inhalation can be either a suspension or a solution and generally contain the active ingredient (s) and a suitable propellant such as a fluorocarbon or hydrogencontaining chlorofluorocarbon or mixtures thereof, particularly hydrofluoroalkanes, e. g. dichlorodifluoromethane, trichlorofluoromethane, dichlorotetra-fluoroethane, especially 1,1, 1, 2-tetrafluoroethane, 1,1, 1,2, 3,3, 3-heptafluoro-n-propane or a mixture thereof. Carbon dioxide or other suitable gas may also be used as propellant. The aerosol composition may be free from excipients other than the propellant or may optionally contain additional formulation excipients well known in the art such as surfactants eg oleic acid or lecithin and cosolvens eg ethanol. Pressurised formulations will generally be retained in a canister (eg an aluminium canister) closed with a valve (eg a metering valve) and fitted into an actuator provided with a mouthpiece.

Medicaments for administration by inhalation desirably have a controlled particle size. The optimum particle size for inhalation into the bronchial system is usually 1-10µ, preferably 2-5µ. Particles having a size above 20µ are generally too large when inhaled to reach the small airways. To achieve these particle sizes the particles of the active ingredient as produced may be size reduced by conventional means eg by micronisation or supercritical fluid techniques. The desired fraction may be separated out by air classification or sieving. Preferably, the particles will be crystalline.

Achieving a high dose reproducibility with micronised powders is difficult because of their poor flowability and extreme agglomeration tendency. To improve the efficiency of dry powder compositions, the particles should be large while in the inhaler, but small when discharged into the respiratory tract. Thus, an excipient such as lactose, manitol or glucose is generally employed. The particle size of the excipient will usually be much greater than the inhaled medicament within the present invention. When the excipient is lactose it will typically be present as milled lactose, preferably crystalline alpha lactose monohydrate.

Pressurized aerosol compositions will generally be filled into canisters fitted with a valve, especially a metering valve. Canisters may optionally be coated with a plastics material e. g. a fluorocarbon polymer as described in W096/32150. Canisters will be fitted into an actuator adapted for buccal delivery.

Typical compositions for nasal delivery include those mentioned above for inhalation and further include non-pressurized compositions in the form of a solution or suspension in an inert vehicle such as water optionally in combination with conventional excipients such as buffers, anti-microbials, mucoadhesive agents, tonicity modifying agents and viscosity modifying agents which may be administered by nasal pump.

Typical dermal and transdermal formulations comprise a conventional aqueous or non-aqueous vehicle, for example a cream, ointment, lotion or paste or are in the form of a medicated plaster, patch or membrane.

The proportions in which (a) the PDE4 inhibitor and (b) the antagonist of M3 muscarinic receptors may be used according to the invention are variable. Active substances (a) and (b) may possibly be present in the form of their solvates or hydrates. Depending on the choice of the compounds (a) and (b), the weight ratios which may be used within the scope of the present invention vary on the basis of the different molecular weights of the various salt forms. The pharmaceutical combinations according to the invention may contain (a) and (b) generally in a ratio by weight (b):(a) ranging from 1: 5 to 500: 1, preferably from 1: 10 to 400: 1.

The weight ratios specified below are based on the compound (b) expressed as 3(R)-(2-hydroxy-2,2-dithien-2-ylacetoxy)-1-(3-phenoxypropyl)-1-azoniabicyclo[2.2.2]octane bromide and the PDE4 inhibitors roflumilast and cilomilast which are particularly preferred according to the invention.

The pharmaceutical combinations according to the invention may contain (a) and (b) in the case of roflumilast, for example, in a ratio by weight (b):(a) ranging from 1: 10 to 300: 1, preferably from 1: 5 to 200: 1, preferably 1: 3 to 150: 1, more preferably from 1: 2 to 100: 1.

The pharmaceutical compositions according to the invention containing the combinations of (a) and (b) are normally administered so that 3(R)-(2-hydroxy-2,2-dithien-2-ylacetoxy)-1-(3-phenoxypropyl)-1-azoniabicyclo[2.2.2]octane bromide and roflumilast are present together in doses of 0,5 to 5000 µg, preferably from 1 to 2000 µg, more preferably from 5 to 1000 µg, better still from 10 to 800 µg per single dose.

For example, without restricting the scope of the invention thereto, combinations in which 3(R)-(2-hydroxy-2,2-dithien-2-ylacetoxy)-1-(3-phenoxypropyl)-1-azoniabicyclo[2.2.2]octane bromide is used as (b) and roflumilast is used as (a), the compositions according to the invention may contain for instance from 20 to 1000 µg of 3(R)-(2-hydroxy-2,2-dithien-2-ylacetoxy)-1-(3-phenoxypropyl)-1-azoniabicyclo[2.2.2]octane bromide and from 5 to 500 µg of roflumilast.

For example, the active substance combinations according to the invention may contain 3(R)-(2-hydroxy-2,2-dithien-2-ylacetoxy)-1-(3-phenoxypropyl)-1-azoniabicyclo[2.2.2]octane bromide and (a) in the case of cilomilast, in a ratio by weight (b):(a) in the range from about 1: 30 to 400: 1, preferably 1: 25 to 200: 1, preferably 1: 20 to 100: 1, more preferably from 1: 15 to 50: 1.

The pharmaceutical compositions according to the invention containing the combinations of (a) and (b) are usually administered so that 3(R)-(2-hydroxy-2,2-dithien-2-ylacetoxy)-1-(3-phenoxypropyl)-1-azoniabicyclo[2.2.2]octane bromide and cilomilast are present together in dosages of 1 to 10000 µg, preferably from 5 to 5000µg, more preferably from 10 to 2000µg, even more preferably from 20 to 800µg per single dose.

For example, without restricting the scope of the invention thereto, combinations in which 3(R)-(2-hydroxy-2,2-dithien-2-ylacetoxy)-1-(3-phenoxypropyl)-1-azoniabicyclo[2.2.2]octane bromide is used as (b) and cilomilast is used as (a), the compositions according to the invention may contain for instance from 5 to 5000 µg of 3(R)-(2-hydroxy-2,2-dithien-2-ylacetoxy)-1-(3-phenoxypropyl)-1-azoniabicyclo[2.2.2]octane bromide and from 15 to 300 µg of cilomilast.

The aforementioned examples of possible doses applicable for the combination according to the invention are to be understood as referring to doses per single application. However, these examples are not to be understood as excluding the possibility of administering the combinations according to the invention multiple times. Depending on the medical need patients may receive also multiple inhalative applications. As an example patients may receive the combinations according to the invention for instance two or three times (e. g. two or three puffs with a powder inhaler, an MDI etc) in the morning of each treatment day. As the aforementioned dose examples are only to be understood as dose examples per single application (i. e. per puff) multiple application of the combinations according to the invention leads to multiple doses of the aforementioned examples. The application of the combinations according to the invention can be for instance once a day, or depending on the duration of action of the anticholinergic agent twice a day, or once every 2 or 3 days.

Preferably the composition is in unit dosage form, for example a tablet, capsule or metered aerosol dose, so that the patient may administer a single dose.

Each dosage unit contains suitably from 20 µg to 1000 µg and preferably from 50 µg to 300 µg of an M3 antagonist according to the invention or a pharmaceutical acceptable salt thereof and 1 µg to 300 µg, and preferably from 5 µg to 100 µg of a PDE4 inhibitor according to the invention.

The amount of each active which is required to achieve a therapeutic effect will, of course, vary with the particular active, the route of administration, the subject under treatment, and the particular disorder or disease being treated.

The active ingredients may be administered from 1 to 6 times a day, sufficient to exhibit the desired activity. Preferably, the active ingredients are administered once or twice a day.

It is contemplated that all active agents would be administered at the same time, or very close in time. Alternatively, one or two actives could be taken in the morning and the other (s) later in the day. Or in another scenario, one or two actives could be taken twice daily and the other (s) once daily, either at the same time as one of the twice-a-day dosing occurred, or separately. Preferably at least two, and more preferably all, of the actives would be taken together at the same time. Preferably, at least two, and more preferably all actives would be administered as an admixture.

The active substance compositions according to the invention are preferably administered in the form of compositions for inhalation delivered with the help of inhalers, especially dry powder inhalers, however, any other form or parenteral or oral application is possible. Here, the application of inhaled compositions embodies the preferred application form, especially in the therapy of obstructive lung diseases or for the treatment of asthma.

The following preparations forms are cited as formulation examples:

### Example 1

| Ingredient | Amount in µg |
|---|---|
| 3(R)-(2-hydroxy-2,2-dithien-2-ylacetoxy)-1-(3-phenoxypropyl)-1-azoniabicyclo[2.2.2]octane bromide | 100 |
| Roflumilast | 5 |
| Lactose | 2.500 |

### Example 2

| Ingredient | Amount in µg |
|---|---|
| 3(R)-(2-hydroxy-2,2-dithien-2-ylacetoxy)-1-(3-phenoxypropyl)-1-azoniabicyclo[2.2.2]octane bromide | 100 |
| Cilomilast | 100 |
| Lactose | 2.500 |

### Example 3

| Ingredient | Amount in µg |
|---|---|
| (3R)-1-phenethyl-3-(9H-xanthene-9-carbonyloxy)-1-azoniabicyclo[2.2.2]octane bromide | 100 |
| Roflumilast | 5 |
| Lactose | 2.500 |

### Example 4

| Ingredient | Amount in µg |
|---|---|
| (3R)-1-phenethyl-3-(9H-xanthene-9-carbonyloxy)-1-azoniabicyclo[2.2.2]octane bromide | 100 |
| Cilomilast | 100 |
| Lactose | 2.500 |

### Example 5

| Ingredient | Amount in µg |
|---|---|
| (3R)-3-[(2S)-2-Cyclopentyl-2-hydroxy-2-thien-2-ylacetoxy]-1-(2-phenoxyethyl)-1-azoniabicyclo[2.2.2]octane bromide | 100 |
| Roflumilast | 5 |
| Lactose | 2.500 |

### Example 7

| Ingredient | Amount in µg |
|---|---|
| (3R)-3-[(2S)-2-Cyclopentyl-2-hydroxy-2-thien-2-ylacetoxy]-1-(2-phenoxyethyl)-1-azoniabicyclo[2.2.2]octane bromide | 100 |
| Cilomilast | 100 |
| Lactose | 2.500 |

### Pharmacological activity

Surprisingly, an unexpectedly beneficial therapeutic effect can be observed in the treatment of inflammatory or obstructive diseases of the respiratory tract if an antimuscarinic of the invention used with one or more PDE4 inhibitors. In view of this effect the pharmaceutical combinations according to the invention can be used in smaller doses than would be the case with the individual compounds used in monotherapy in the usual way. This reduces unwanted side effects such as may occur when PDE4 inhibitorsare administered, for example.

Consequently, the combinations of the invention possess therapeutically advantageous properties, which make them particularly suitable for the treatment of respiratory diseases in all kind of patients.

## Claims

1. A combination which comprises (a) a PDE4 inhibitor and (b) an antagonist of M3 muscarinic receptors which is 3(R)-(2-hydroxy-2,2-dithien-2-ylacetoxy)-1-(3-phenoxypropyl)-1-azoniabicyclo[2.2.2]octane, in the form of a salt having an anion X, which is a pharmaceutically acceptable anion of a mono or polyvalent acid.

2. A combination according to claim 1 wherein the antagonist of M3 muscarinic receptor (b) is 3(R)-(2-hydroxy-2,2-dithien-2-ylacetoxy)-1-(3-phenoxypropyl)-1-azoniabicyclo[2.2.2]octane bromide.

3. A combination according to claim 1 or 2 wherein the PDE4 inhibitor is selected from the group comprising : theophylline, drotaverine hydrochloride, cilomilast, roflumilast, denbufylline, rolipram, tetomilast, enprofylline, arofylline, cipamfylline, tofimilast, filaminast, piclamilast, (R)-(+)-4-[2-(3-cyclopentyloxy-4-methoxyphenyl)-2-phenylethyl]pyridine, mesopram, N-(3,5-dichloro-4-pyridinyl)-2-[1-(4-fluorobenzyl)-5-hydroxy-1H-indol-3-yl]-2-oxoacetamide, CDC-801 (ex. Celgene), CC-1088 (ex. Celgene), Lirimilast, ONO-6126 (ex. Ono), CC-10004 (ex. Celgene) and MN-001 (ex. Kyorin), optionally in the form of their racemates, their enantiomers, their diastereomers and mixtures thereof, and optionally their pharmacologically-compatible acid addition salts.

4. A combination according to claim 3 wherein the PDE4 inhibitor is selected from the group comprising cilomilast, roflumilast, denbufylline and tetomilast optionally in the form of their racemates, their enantiomers, their diastereomers and mixtures thereof, and optionally their pharmacologically-compatible acid addition salts.

5. A combination according to claim 4 wherein the PDE4 inhibitor is roflumilast.

6. A combination according to claim 4 wherein the PDE4 inhibitor is cilomilast.

7. A combination according to any one of the preceding claims **characterised in that** the active ingredients (a) and (b) form part of a single pharmaceutical composition.

8. A combination according to any one of the preceding claims which further comprises (c) another active compound selected from: (a) β2-agonists, (b) cortiocosteroids, (c) leukotriene D4 antagonists, (d) inhibitors of egfr-kinase, (e) p38 kinase inhibitors and (f) NK1 receptor agonists.

9. A combination according to claim 8 wherein the active compound (c) is selected from the group consisting of (a) β2-agonists and (b) cortiocosteroids.

10. Use of (a) a PDE4 inhibitor as defined in any one of claims 1 and 3 to 6 and (b) an antagonist of M3 muscarinic receptors as defined in claim 1 or 2, for the preparation of a medicament for simultaneous, concurrent, separate or sequential use in the treatment of a respiratory disease which responds to M3 antagonism in a patient.

11. Use according to claim 10 wherein the respiratory disease is asthma or chronic obstructive pulmonary disease (COPD).

12. A product comprising (a) a PDE4 inhibitor as defined in any one of claims 1 and 3 to 6 and (b) an antagonist of M3 muscarinic receptors as defined in claim 1 or 2, as a combined preparation for simultaneous, concurrent, separate or sequential use in the treatment of a patient suffering from or susceptible to a respiratory disease which responds to M3 antagonism.

13. A product according to claim 12, which further comprises an active compound (c), as defined in claim 8 or 9.

14. A kit of parts comprising (b) an antagonist of M3 muscarinic receptors as defined in claim 1 or 2 together with instructions for simultaneous, concurrent, separate or sequential use in combination with (a) a PDE4 inhibitor as defined in any one of claims 1 and 3 to 6 for the treatment of a patient suffering from or susceptible to a respiratory disease as defined in claim 10 or 11.

15. A kit according to claim 14, which further comprises an active compound (c), as defined in claim 8 or 9.

16. A package comprising (b) an antagonist of M3 muscarinic receptors as defined in claim 1 or 2 and (a) a PDE4 inhibitor as defined in any one of claims 1 and 3 to 6, for the simultaneous, concurrent, separate or sequential use in the treatment of a respiratory disease as defined in claim 10 or 11.

17. A package according to claim 16, which further comprises an active compound (c), as defined in claim 8 or 9.

18. Use of (b) an antagonist of M3 muscarinic receptors as defined in claim 1 or 2 for the preparation of a medicament, for simultaneous, concurrent, separate or sequential use in combination with (a) a PDE4 inhibitor as defined in any one of claims 1 and 3 to 6 for the treatment of a respiratory disease as defined in claim 10 or 11.

19. Use of (a) a PDE4 inhibitor as defined in any one of claims 1 and 3 to 6 for the preparation of a medicament, for simultaneous, concurrent, separate or sequential use in combination with (b) an antagonist of M3 muscarinic receptors as defined in claim 1 or 2 for the treatment of a respiratory disease as defined in claim 10 or 11.

## Patentansprüche

1. Kombination, welche (a) einen PDE4-Inhibitor und (b) einen Antagonisten von muskarinischen M3-Rezeptoren, welcher 3(R)-(2-Hydroxy-2,2-dithien-2-ylacetoxy)-1-(3-phenoxypropyl)-1-azoniabicyclo[2.2.2]octan ist, in der Form eines Salzes, das ein Anion X hat, welches ein pharmazeutisch annehmbares Anion einer ein- oder mehrwertigen Säure ist, umfasst.

2. Kombination nach Anspruch 1, wobei der Antagonist von muskarinischem M3-Rezeptor (b) 3(R)-(2-Hydroxy-2,2-dithien-2-ylacetoxy)-1-(3-phenoxypropyl)-1-azoniabicyclo[2.2.2]octan-Bromid ist.

3. Kombination nach Anspruch 1 oder 2, wobei der PDE4-Inhibitor ausgewählt ist aus der Gruppe, bestehend aus Theophyllin, Drotaverin-Hydrochlorid, Cilomilast, Roflumilast, Denbufyllin, Rolipram, Tetomilast, Enprofyllin, Arofyllin, Cipamfyllin, Tofimilast, Filaminast, Piclamilast, (R)-(+)-4-[2-(6-Cyclopentyloxy-4-methoxyphenyl)-2-phenylethyl]-pyridin, Mesopram, N-(3,5-Dichlor-4-pyridinyl)-2-[1-(4-fluorbenzyl)-5-hydroxy-1H-indol-3-yl]-2-oxoacetamid, CDC-801 (von Celgene), CC-1088 (von Celgene), Lirimilast, ONO-6126 (von Ono), CC-10004 (von Celgene) und MN-001 (von Kyorin), gegebenenfalls in der Form ihrer Racemate, ihrer Enantiomeren, ihrer Diastereomeren und Gemischen davon, und gegebenenfalls ihren pharmakologisch verträglichen Säureadditionssalzen.

4. Kombination nach Anspruch 3, wobei der PDE4-Inhibitor ausgewählt ist aus der Gruppe, umfassend Cilomilast, Roflumilast, Denbufyllin und Tetomilast, gegebenenfalls in der Form ihrer Racemate, ihrer Enantiomeren, ihrer Diastereomeren und Gemischen davon, und gegebenenfalls ihre pharmakologisch verträglichen Säureadditionssalze.

5. Kombination nach Anspruch 4, wobei der PDE4-Inhibitor Roflumilast ist.

6. Kombination nach Anspruch 4, wobei der PDE4-Inhibitor Cilomilast ist.

7. Kombination nach einem der vorangehenden Ansprüche, **dadurch gekennzeichnet, dass** die aktiven Ingredienzien (a) und (b) Bestandteil einer einzelnen pharmazeutischen Zusammensetzung sind.

8. Kombination nach einem der vorangehenden Ansprüche, die außerdem (c) eine andere aktive Verbindung, ausgewählt aus: (a) β2-Agonisten, (b) Corticosteroiden, (c) Leukotrien-D4-Antagonisten, (d) Inhibitoren von egfr-Kinase, (e) p38-Kinase-Inhibitoren und (f) NK1-Rezeptor-Agonisten, umfasst.

9. Kombination nach Anspruch 8, wobei die aktive Verbindung (c) ausgewählt ist aus der Gruppe, bestehend aus (a) β2-Agonisten und (b) Corticosteroiden.

10. Verwendung (a) eines PDE4-Inhibitors, wie er in einem der Ansprüche 1 und 3 bis 6 definiert ist, und (b) eines Antagonisten von muskarinischen M3-Rezeptoren, wie er in Anspruch 1 oder 2 definiert ist, für die Herstellung eines Medikaments zur gleichzeitigen, gleichlaufenden, getrennten oder sequentiellen Verwendung bei der Behandlung einer Atemwegserkrankung, die auf M3-Antagonismus anspricht, bei einem Patienten.

11. Verwendung nach Anspruch 10, wobei die Atemwegserkrankung Asthma oder chronisch-obstruktive Lungenerkrankung (COPD) ist.

12. Produkt, umfassend (a) einen PDE4-Inhibitor, wie er in einem der Ansprüche 1 und 3 bis 6 definiert ist, und (b) einen Antagonisten von muskarinischen M3-Rezeptoren, wie er in Anspruch 1 oder 2 definiert ist, als ein kombiniertes Präparat zur gleichzeitigen, gleichlaufenden, getrennten oder sequentiellen Verwendung bei der Behandlung eines Patienten, der an einer Atemwegserkrankung, wie sie in Anspruch 10 oder 11 definiert ist, leidet oder dafür anfällig ist.

13. Produkt nach Anspruch 12, das außerdem eine aktive Verbindung (c), wie sie in Anspruch 8 oder 9 definiert ist, umfasst.

14. Kit von Komponenten, umfassend (b) einen Antagonisten von muskarinischen M3-Rezeptoren, wie er in Anspruch 1 oder 2 definiert ist, zusammen mit Instruktionen zur gleichzeitigen, gleichlaufenden, getrennten oder sequentiellen Verwendung in Kombination mit (a) einem PDE4-Inhibitor, wie er in einem der Ansprüche 1 und 3 bis 6 definiert ist, für die Behandlung eines Patienten, der an einer Atemwegserkrankung, wie sie in Anspruch 10 oder 11 definiert ist, leidet oder dafür anfällig ist.

15. Kit nach Anspruch 14, der außerdem eine aktive Verbindung (c), wie sie in Anspruch 8 oder 9 definiert ist, umfasst.

16. Packung, umfassend (b) einen Antagonisten von muskarinischen M3-Rezeptoren, wie er in Anspruch 1 oder 2 definiert ist, und (a) einen PDE4-Inhibitor, wie er in einem der Ansprüche 1 und 3 bis 6 definiert ist, für die gleichzeitige, gleichlaufende, getrennte oder sequentielle Verwendung bei der Behandlung einer Atemwegserkrankung, wie sie in Anspruch 10 oder 11 definiert ist.

17. Packung nach Anspruch 16, die außerdem eine aktive Verbindung (c), wie sie in Anspruch 8 oder 9 definiert ist, umfasst.

18. Verwendung (b) eines Antagonisten von muskarinischen M3-Rezeptoren, wie er in Anspruch 1 oder 2 definiert ist, für die Herstellung eines Medikaments zur gleichzeitigen, gleichlaufenden, getrennten oder sequentiellen Verwendung in Kombination mit (a) einem PDE4-Inhibitor, wie er in einem der Ansprüche 1 und 2 bis 6 definiert ist, für die Behandlung einer Atemwegserkrankung, wie sie in Anspruch 10 oder 11 definiert ist.

19. Verwendung (a) eines PDE4-Inhibitors, wie er in einem der Ansprüche 1 und 3 bis 6 definiert ist, für die Herstellung eines Medikaments zur gleichzeitigen, gleichlaufenden, getrennten oder sequentiellen Verwendung in Kombination mit (b) einem Antagonisten von muskarinischen M3-Rezeptoren, wie er in Anspruch 1 oder 2 definiert ist, für die Behandlung einer Atemwegserkrankung, wie sie in Anspruch 10 oder 11 definiert ist.

## Revendications

1. Combinaison qui comprend (a) un inhibiteur de PDE4 et (b) un antagoniste de récepteurs muscariniques M3 qui est 3(R)-(2-hydroxy-2,2-dithiène-2-ylacétoxy)-1-(3-phénoxypropyl)-1-azoniabicyclo[2.2.2]octane, sous la forme d'un sel ayant un anion X, qui est un anion pharmaceutiquement acceptable d'un acide mono ou polyvalent.

2. Combinaison selon la revendication 1, dans laquelle l'antagoniste de récepteur muscarinique M3 (b) est le bromure de 3(R)-(2-hydroxy-2,2-dithiène-2-ylacétoxy)-1-(3-phénoxypropyl)-1-azoniabicyclo[2.2.2]octane.

3. Combinaison selon la revendication 1 ou 2 dans laquelle l'inhibiteur de PDE4 est choisi parmi le groupe comprenant : théophylline, chlorhydrate de drotavérine, cilomilast, roflumilast, denbufylline, rolipram, tétomilast, enprofylline, arofylline, cipamfylline, tofimilast, filaminast, piclamilast, (R)-(+)-4-[2-(3-cyclopentyloxy-4-méthoxyphényl)-2-phényléthyl]pyridine, mésopram, N-(3,5-dichloro-4-pyridinyl)-2-[1-(4-fluorobenzyl)-5-hydroxy-1H-indol-3-yl]-2-oxoacétamide, CDC-801 (ex. Celgene), CC-1088 (ex. Celgene), Lirimilast, ONO-6126 (ex. Ono), CC-10004 (ex. Celgene) et MN-001 (ex. Kyorin), éventuellement sous la forme de leurs racémates, leurs énantiomères, leurs diastéréomères et des mélanges de ceux-ci, et éventuellement leurs sels d'addition d'acide pharmacologiquement compatibles.

4. Combinaison selon la revendication 3 dans laquelle l'inhibiteur de PDE4 est choisi parmi le groupe comprenant cilomilast, roflumilast, denbufylline et tétomilast éventuellement sous la forme de leurs racémates, leurs énantiomères, leurs diastéréomères et des mélanges de ceux-ci, et éventuellement leurs sels d'addition d'acide pharmacologiquement compatibles.

5. Combinaison selon la revendication 4 dans laquelle l'inhibiteur de PDE4 est le roflumilast.

6. Combinaison selon la revendication 4 dans laquelle l'inhibiteur de PDE4 est le cilomilast.

7. Combinaison selon l'une quelconque des revendications précédentes **caractérisée en ce que** les ingrédients actifs (a) et (b) font partie d'une seule composition pharmaceutique.

8. Combinaison selon l'une quelconque des revendications précédentes qui comprend en outre (c) un autre principe actif choisi parmi : (a) des agonistes β2, (b) des corticostéroïdes, (c) des antagonistes de leucotriène D4, (d) des inhibiteurs de la kinase egfr, (e) des inhibiteurs de la kinase p38 et (f) des agonistes de récepteurs NK1.

9. Combinaison selon la revendication 8 dans laquelle le principe actif (c) est choisi parmi le groupe constitué (a) d'agonistes β2 et (b) de corticostéroïdes.

10. Utilisation de (a) un inhibiteur de PDE4 tel que défini dans l'une quelconque des revendications 1 et 3 à 6 et (b) un antagoniste de récepteurs muscariniques M3 tel que défini à la revendication 1 ou 2, pour la préparation d'un médicament pour une utilisation simultanée, concurrente, séparée ou séquentielle dans le traitement d'une maladie respiratoire qui répond à un antagonisme M3 chez un patient.

11. Utilisation selon la revendication 10 dans laquelle la maladie respiratoire est l'asthme ou une maladie pulmonaire obstructive chronique (MPOC).

12. Produit comprenant (a) un inhibiteur de PDE4 tel que défini dans l'une quelconque des revendications 1 et 3 à 6 et (b) un antagoniste de récepteurs muscariniques M3 tel que défini à la revendication 1 ou 2, en tant que préparation combinée pour une utilisation simultanée, concurrente, séparée ou séquentielle dans le traitement d'un patient souffrant de, ou sensible à, une maladie respiratoire qui répond à un antagonisme M3.

13. Produit selon la revendication 12, qui comprend en outre un principe actif (c), tel que défini à la revendication 8 ou 9.

14. Kit de parties comprenant (b) un antagoniste de récepteurs muscariniques M3 tel que défini à la revendication 1 ou 2 ainsi que des instructions pour une utilisation simultanée, concurrente, séparée ou séquentielle en combinaison avec (a) un inhibiteur de PDE4 tel que défini dans l'une quelconque des revendications 1 et 3 à 6 pour le traitement d'un patient souffrant de, ou sensible, à une maladie respiratoire telle que définie à la revendication 10 ou 11.

15. Kit selon la revendication 14, qui comprend en outre un principe actif (c), tel que défini à la revendication 8 ou 9.

16. Conditionnement comprenant (b) un antagoniste de récepteurs muscariniques M3 tel que défini à la revendication 1 ou 2 et (a) un inhibiteur de PDE4 tel que défini dans l'une quelconque des revendications 1 et 3 à 6, pour l'utilisation simultanée, concurrente, séparée ou séquentielle dans le traitement d'une maladie respiratoire telle que définie à la revendication 10 ou 11.

17. Conditionnement selon la revendication 16, qui comprend en outre un principe actif (c), tel que défini à la revendication 8 ou 9.

18. Utilisation de (b) un antagoniste de récepteurs muscariniques M3 tel que défini à la revendication 1 ou 2 pour la préparation d'un médicament pour une utilisation simultanée, concurrente, séparée ou séquentielle en combinaison avec (a) un inhibiteur de PDE4 tel que défini dans l'une quelconque des revendications 1 et 3 à 6 pour le traitement d'une maladie respiratoire telle que définie à la revendication 10 ou 11.

19. Utilisation de (a) un inhibiteur de PDE4 tel que défini dans l'une quelconque des revendications 1 et 3 à 6 pour la préparation d'un médicament destiné à un usage simultané, concurrent, séparé ou séquentiel en combinaison avec (b) un antagoniste de récepteurs muscariniques M3 tel que défini à la revendication 1 ou 2 pour le traitement d'une maladie respiratoire telle que définie à la revendication 10 ou 11.
